# FASCICULE DE BREVET EUROPEEN

(11) **EP 1 935 406 B1**
(45) Date de publication et mention de la délivrance du brevet: **29.04.2015**
(21) Numéro de dépôt: 07123854.7
(22) Date de dépôt: 20.12.2007
(51) Int. Cl.: A61K 9/00, A61K 31/138, A61K 9/20

(54) **Formulations pharmaceutiques dispersibles contenant de la fluoxétine**
Pharmazeutische Sprühformulierungen, die Fluoxetin enthalten
Dispersible pharmaceutical formulations containing fluoxetine

(30) Priorité: 20.12.2006 FR 0655708
(43) Date de publication de la demande: 25.06.2008
(73) Titulaire: Substipharm Developpement, 75016 Paris (FR)
(72) Inventeur: Berthier, César, 75116 PARIS (FR)
(74) Mandataire: Regimbeau

(56) Documents cités:
- WO-A-00/27357
- WO-A-2004/000197
- WO-A-2004/100857

## Description

L'invention a pour objet des formulations pharmaceutiques contenant de la fluoxétine, ou un sel d'addition acide de celle-ci, appropriées pour la fabrication de comprimés dispersibles.

La fluoxétine, ou N-méthyl-3-(p-trifluorométhylphenoxy)-3-phénylpropylamine est un agent antidépresseur (DE 2 500 110, US 4,314,081), en particulier un inhibiteur sélectif de la recapture de la sérotonine. Ses indications thérapeutiques sont particulièrement le traitement des épisodes dépressifs majeurs, des troubles obsessionnels compulsifs, de la boulimie (diminution de la fréquence des crises de boulimie et des vomissements ou prise de laxatifs). La fluoxétine est le plus fréquemment utilisée sous la forme de son sel de chlorhydrate.

Actuellement, la fluoxétine, en particulier son sel de chlorhydrate, est disponible sous la formes de gélules, de solutions buvables et de comprimées dispersibles.

Ainsi, la demande de brevet EP 693 281 décrit une formulation pharmaceutique appropriée pour la préparation de comprimés dispersibles, par compression directe, constituée de fluoxétine, ou d'un sel d'addition de celle-ci, et d'excipients et co-adjuvants appropriés. Ces excipients et co-adjuvants appropriés contiennent notamment :
- un délitant (disintégrant, en anglais), choisi dans le groupe comprenant le glycolate d'amidon de sodium, des dérivés polymères d'acide acrylique et la crospovidone,
- un diluant, choisi dans le groupe comprenant de la cellulose microcristalline, du lactose, de l'hydroxypropylcellulose (HPC), de l'amidon prégélatinisé, de l'amidon apte à un écoulement à sec, ainsi que leurs mélanges,
- un anti-adhérant.

La formulation peut en outre comprendre, à titre d'excipients et de co-adjuvants,
- un lubrifiant (fumarate stéarylique de sodium)
- des édulcorants, et
- des arômes.

Les comprimés dispersibles sont des formes pharmaceutiques solides, destinées à l'administration par voie orale. Conformément aux pharmacopées, notamment la pharmacopée européenne, ils doivent se désagréger dans de l'eau à 15°C-25°C en moins de 3 minutes et former dans l'eau des dispersions uniformes. Par ailleurs, la suspension obtenue après dissolution totale de deux comprimés dans 100 mL d'eau doit passer à travers un tamis de 710µm.

La compression directe ne comprend pas d'étape de granulation préalable à la compression et permet un gain de temps considérable. Etant donné que la fluoxétine ou ses sels d'addition, comme la plupart des principes actifs, possède une mauvaise aptitude à la compressibilité, elle doit être mélangée à des excipients, qui sont directement compressibles et qui sont compatibles avec le principe actif, pour pouvoir subir une compression directe.

La compression directe est effectuée sur des machines rotatives à haute vitesse. La trémie d'alimentation fonctionne en général par gravité et est sensible à l'agglomération des poudres ou à leur prise en masse. La rhéologie du mélange de poudres à comprimer est donc un facteur à prendre en considération pour garantir l'uniformité de poids des comprimés et l'uniformité de leur contenu.

Un autre inconvénient de la technique de compression directe provient du risque de séparation des poudres ou « démélange ». Ce démélange conduit à des comprimés non homogènes en composition.

Ainsi, en utilisant la technique de compression directe, des risques de mauvaise distribution du principe actif dans les excipients et/ou de séparation du principe actif et des excipients conduisant à un comprimé non homogène existent. Par ailleurs, on peut rencontrer des difficultés liées à une mauvaise fluidité du mélange de poudres ou des problèmes de collage, de grippage ou de décalotage, rendant difficile l'éjection du comprimé.

Un excipient, pour être utilisé en compression directe, doit posséder une bonne coulabilité, ne doit pas s'agglomérer de façon spontanée, doit former un comprimé de bonne tenue mécanique ou cohésif sous l'effet d'une force de compression raisonnable et doit permettre un délitement en un temps adapté. De nombreux diluants et liants directement compressibles ont été développés. Certains excipients pour compression directe sont onéreux car ils nécessitent des procédés de préparation élaborés ou l'adjonction de nombreux additifs.

L'homme du métier est toujours à la recherche de formulations alternatives pour comprimés dispersibles qui peuvent être soumises à une compression directe. Par ailleurs, il est désireux de s'émanciper de l'étape préalable de revêtement de la fluoxétine ou l'un de ses sels, ce qui toutefois augmente les risques d'hétérogénéité de la formulation et de démélange et requiert par conséquent un choix stratégique des excipients. Bien entendu, l'homme du métier souhaite en outre éviter et/ou limiter l'utilisation d'excipients à effet notoire.

Les inventeurs ont constaté que, d'une manière surprenante, l'utilisation de deux sucres de granulométrie différente permettait d'assurer à la fois une bonne homogénéité de répartition du principe actif (fluoxétine ou un de ses sels d'addition) et une bonne compressibilité de la formulation. Le choix des autres excipients est alors plus flexible. Cette formulation permet l'emploi de la fluoxétine sans traitement préalable, c'est-à-dire sans étape préalable d'enrobage du principe actif.

La demande internationale WO 2004/000197 décrit des formulations de fluoxétine pouvant comprendre deux sucres de granulométries différentes. Toutefois, ces formulations sont destinées à la fabrication de comprimés orodipersibles (dispersibles en bouche en moins de 40s, pas de dispersion préalable dans une solution aqueuse) et non à la fabrication de comprimés dispersibles. Les exigences techniques ne sont pas les même d'un type de formulation à l'autre. Par ailleurs, la fluoxétine est sous forme de microsphères masquant le goût, ce qui permet également d'assurer une granulométrie adaptée à ce type de formulation mais induit des coûts de fabrication plus élevés (par rapport à l'utilisation de la fluoxétine telle quelle, sans prétraitement).

La demande internationale WO 00/27357 décrit un comprimé orodispersible pouvant comprendre outre un principe actif (la fluoxétine n'est pas citée) deux sucres de granulométrie différente (en particulier deux mannitol : Pearlitol® 400 et Advantose® 100). Toutefois, cette formulation n'est pas destinée à la préparation de comprimés dispersibles, pour lesquels les exigences techniques sont différentes. Par ailleurs, le principe actif est préalablement revêtu, ce qui permet d'assurer une granulométrie adaptée à ce type de formulation mais induit des coûts de fabrication plus élevés (par rapport à l'utilisation du principe actif tel quel, sans prétraitement).

L'objet de l'invention est donc une formulation pharmaceutique appropriée pour la préparation de comprimés dispersibles, par compression directe, constitués de fluoxétine ou d'un sel d'addition acide de celle-ci, à titre de principe actif, en une quantité de 4% à 7,5% en poids par rapport au poids total de la formulation, et d'excipients et coadjuvants appropriés, dont au moins un diluant, un délitant et un lubrifiant, caractérisée en ce que ledit diluant est un mélange de
- sucres de faible granulométrie, avec une taille moyenne de particules comprise entre 100 µm et 250 µm, et
- sucres de granulométrie moyenne, avec une taille moyenne de particules comprise entre 300 µm et 600 µm,
lesdits sucres étant choisis dans le groupe constitué par le mannitol, le lactose et leurs mélanges.

Dans le cadre de la présente invention, on entend désigner par l'expression « comprimé dispersible » un comprimé non enrobé ou pelliculé qui est destiné à être dispersé, sans toutefois être dissous, dans de l'eau avant son administration sous forme de suspension. Le temps de désagrégation dans l'eau doit être inférieur à 3 minutes. Cette expression n'entend pas inclure les comprimés orodipersibles (comprimés destinés à être dispersés directement dans la bouche, avec un temps de désagrégation généralement admis de : inférieur à 1 minute).

Selon l'invention, la fluoxétine, ou de l'un de ses sels est utilisée telle quelle, non revêtue (c'est-à-dire n'ayant pas subi d'étape préalable de revêtement et/ou de mise en forme ou granulation telle que l'obtention de microsphères).

Dans le cadre de la présente invention, on utilise de préférence
- d'une part des particules de sucre présentant une grande surface spécifique et une faible granulométrie, pour assurer une bonne répartition du principe actif autour des granulés,
- d'autre part des particules de sucre de granulométrie comprise entre 300 µm et 600 µm, plus avantageusement comprise entre 300 µm et 450 µm, encore plus avantageusement comprise entre 320 µm et 400 µm, pour assurer une bonne compressibilité de la formulation.

En particulier, les particules de sucres de faible granulométrie ont une surface spécifique supérieure à 0,90 m²/g.

Pour s'assurer que les particules de sucre de faible granulométrie présentent une grande surface spécifique, on utilisera de préférence des particules obtenues par atomisation en lit fluidisé.

Les particules de sucre de granulométrie comprise entre 300 µm et 600 µm pourront, quant à elles, être obtenues par extrusion.

Dans le cadre de la présente invention, pour les sucres de granulométrie moyenne, on préfère utiliser des particules de sucre ayant une granulométrie comprise entre 300 µm et 450 µm, plus avantageusement comprise entre 320 µm et 400 µm. En effet, afin d'assurer une bonne compression de la formulation, il faut éviter une trop grande disparité entre les granulométries des différents excipients.

Selon une variante avantageuse de l'invention, le diluant est un mélange de
- mannitol avec une taille moyenne de particules comprise entre 150 µm et 220 µm, obtenu par atomisation en lit fluidisé (le mannitol est un excipient n'ayant aucun effet notoire)
- mannitol avec une taille moyenne de particules comprise entre 320 µm et 400 µm.

En particulier, le diluant est un mélange de PEARLITOL^{®} 200 SD et de PEARLITOL^{®} 400 DC (marques déposées par la société ROQUETTE).

Le PEARLITOL^{®} 200 SD ou Mannitol 200 SD désigne des particules de mannitol obtenues par atomisation, avec une taille moyenne de particules de 180 µm ; 10% maximum de particules ont une granulométrie supérieure à 315 µm et 90% minimum des particules ont une granulométrie supérieure à 75 µm (granulométrie mesurée par laser).

Le PEARLITOL^{®} 400 DC ou Mannitol 400 DC désigne des particules de mannitol obtenues par extrusion, avec une taille moyenne de particules de 360 µm ; 20% maximum de particules ont une granulométrie supérieure à 500 µm et 85% minimum des particules ont une granulométrie supérieure à 100 µm (granulométrie mesurée sur tamis).

Toutefois, on pourrait utiliser du PEARLITOL^{®} 500DC à la place du PEARLITOL^{®} 400DC.

Le PEARLITOL^{®} 500 DC ou Mannitol 500 DC désigne des particules de mannitol obtenues par extrusion, avec une taille moyenne de particules de 520 µm.

Dans la formulation, la teneur totale en diluant est avantageusement comprise entre 75% et 90% en poids, plus avantageusement entre 80% et 90% en poids, par rapport au poids total de la formulation.

Le ratio massique sucres de faible granulométrie : sucres de granulométrie moyenne est avantageusement de 1.

Un délitant est une substance dont le rôle est d'accélérer la désagrégation du comprimé, donc la dispersion du principe actif dans l'eau. Dans le cadre de la présente invention, tout délitant pourra être utilisé. On peut ainsi citer, comme exemple de délitant, l'acide alginique, le benzoate de sodium, la carboxyméthylcellulose calcique, la carboxyméthylcellulose sodique, la carboxyméthylcellulose sodique réticulée (en anglais : croscarmellose sodium), la crospovidone, le dibéhénate de glycérol, la gomme de guar, le silicate d'aluminium et de magnésium, l'éther méthylique de cellulose (méthylcellulose), la polacrilin-potassium, le glycolate d'amidon de sodium et des dérivés polymères d'acide acrylique.

Selon une variante avantageuse de l'invention, le délitant est choisi dans le groupe constitué par la carboxyméthylcellulose sodique réticulée, le glycolate d'amidon sodique, et la crospovidone.

Dans la formulation, la teneur en délitant est avantageusement comprise entre 0,5% et 5% en poids, par rapport au poids total de la formulation.

Lorsque le délitant est la carboxyméthylcellulose sodique réticulée, sa teneur en poids dans la formulation, par rapport au poids total de ladite formulation, varie avantageusement de 0,5% à 2%, plus avantageusement de 0,8% à 1,5%.

Lorsque le délitant est la crospovidone, sa teneur en poids dans la formulation, par rapport au poids total de ladite formulation, varie avantageusement de 1% à 5%, plus avantageusement de 2% à 5%.

Lorsque le délitant est le glycolate d'amidon sodique, sa teneur en poids dans la formulation, par rapport au poids total de ladite formulation, varie avantageusement de 1 % à 5%, plus avantageusement de 2% à 5%.

Un lubrifiant est un excipient qui permet de diminuer le frottement entre les particules dans le comprimé. En outre, le lubrifiant facilite la compression et l'éjection des comprimés. Dans le cadre de la présente invention, tout lubrifiant pourra être utilisé. On peut ainsi citer, comme exemple de lubrifiant, le stéarate de calcium, l'huile végétale hydrogénée de type I, le stéarate de magnésium, l'acide stéarique, le talc et le stéarate de zinc.

Selon une variante avantageuse de l'invention, le lubrifiant est choisi dans le groupe constitué par le stéarate de magnésium et le talc.

La teneur en lubrifiant, dans la formulation, est avantageusement comprise entre 0,25% et 5% en poids, par rapport au poids total de ladite formulation.

Lorsque le lubrifiant est le stéarate de magnésium, sa teneur en poids dans la formulation, par rapport au poids total de ladite formulation, varie avantageusement de 0,5% à 3%, plus avantageusement de 1% à 2%.

Lorsque le lubrifiant est le talc, sa teneur en poids dans la formulation, par rapport au poids total de ladite formulation, varie avantageusement de 1 % à 5%.

La formulation pharmaceutique selon l'invention peut en outre comprendre, à titre d'excipients et coadjuvants appropriés, des édulcorants et des arômes.

En particulier, la formulation selon l'invention comprend, à titre d'arôme, des arômes de menthe poivrée et des arômes d'anis.

La formulation selon l'invention comprend avantageusement, à titre d'édulcorant, la saccharine sodique.

Selon une variante avantageuse de l'invention, dans la formulation, les excipients et co-adjuvants sont constitués :
- d'un mélange de:
   i. mannitol ayant une taille moyenne de particules comprise entre 150 µm et 220 µm, lesdites particules ayant de préférence une surface spécifique supérieure à 0,90 m²/g (elles peuvent être obtenues par atomisation en lit fluidisé)
   ii. mannitol ayant une taille moyenne de particules comprise entre 320 µm et 450 µm,
- de la carboxyméthylcellulose sodique réticulée,
- du stéarate de magnésium,
- des arômes et édulcorants.

On constate que cette formulation est d'une grande simplicité et qu'elle n'implique pas d'excipients onéreux.

En effet, les excipients utilisés sont tous des excipients classiquement utilisés en formulation. Ils n'imposent pas de conditions particulières de manipulation, qui vont au-delà des connaissances techniques générales d'un ingénieur en formulation.

Ainsi, par exemple, le lubrifiant utilisé peut simplement être le stéarate de magnésium. Contrairement à la formulation décrite dans le brevet EP 693 281, il n'est pas nécessaire d'utiliser des lubrifiants onéreux tels que le stéaryl fumarate.

Par ailleurs, l'utilisation d'agents anti-adhérents s'est révélée non nécessaire.

L'invention a également pour objet les comprimés dispersibles obtenus par compression directe de la formulation selon l'invention.

Selon une variante avantageuse de l'invention, la fluoxétine ou une de ses sels d'addition est préalablement mélangée avec les particules de sucre de faible granulométrie avant ajout des autres excipients.

Lors de la compression directe, aucun phénomène de collage, grippage ou décalotage n'est observé. Par ailleurs, les comprimés obtenus sont de composition homogène.

Ces comprimés présentent les caractéristiques suivantes :
- Temps de désagrégation dans une eau à 15°C-25°C : inférieur à 3 min
- Finesse de la suspension : passage au travers du tamis de 710µm

Ces comprimés sont, en accord avec les différentes Pharmacopées, en particulier avec la Pharmacopée Européenne, qualifiés de comprimés dispersibles.

Les comprimés dispersibles obtenus sont solides, appropriés pour une utilisation par voie orale, d'apparence uniforme, et ont une dureté suffisante pour pouvoir supporter les éventuels chocs lors du transport et du stockage et l'extraction du conditionnement.

Les comprimés dispersibles obtenus ont avantageusement une dureté de 70N±20N. Leur friabilité est avantageusement inférieure à 1%, en accord avec la pharmacopée Européenne.

Par ailleurs, la solution obtenue après dispersion des comprimés est parfaitement limpide, plus limpide que la référence.

Les exemples qui suivent illustrent l'invention.

### Exemple 1 : Comprimé dispersible

La formulation suivante a été soumise à une compression directe :

**Tableau 1**

| Composés | Formulation | |
|---|---|---|
| | % | mg/comprimé |
| **Principe actif** | | |
| Chlorhydrate de fluoxétine | 7,22 | 22,37 |
| exprimé en tant que fluoxétine | | 20,00 |
| **Excipients** | | |
| Mannitol 200SD¹ | 43,35 | 134,40 |
| Mannitol 400 DC² | 42,26 | 131,00 |
| Carboxyméthylcellulose sodique réticulée | 0,97 | 3,00 |
| Stéarate de magnésium | 1,50 | 4,65 |
| Saccharine sodique | 3,61 | 11,20 |
| Arôme anis | 0,73 | 11,20 |
| Arôme menthe poivrée | 0,36 | 1,12 |
| **Total** | 100,00 | 310,00 |

| | | |
|---|---|---|
| ¹ PEARLITOL^{®} 200 SD commercialisé par la société Roquette ² PEARLITOL^{®} 400 DC commercialisé par la société Roquette | | |

Les comprimés obtenus présentent les propriétés suivantes :
- Dureté : 70N±20N
- Friabilité : conforme Ph.Eur. <1%
- Temps de délitement < 3 min
- Finesse de la suspension : passe au travers du tamis de 710µm

Dureté: cet essai est destiné à déterminer, dans des conditions définies, la résistance à la rupture des comprimés, mesurée par la force nécessaire pour provoquer leur rupture par compression diamétrale.

Friabilité: cet essai est destiné à déterminer, dans des conditions définies, la friabilité des comprimés non enrobés, c'est-à-dire le phénomène par lequel la surface des comprimés est endommagée ou présente des signes d'abrasion ou de rupture sous l'effet de chocs mécaniques ou d'une attrition.

### Exemple 2 : mesures de turbidité

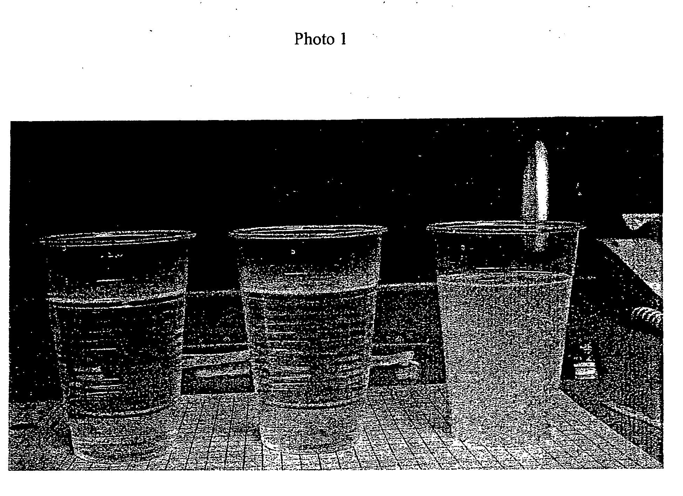

La solution obtenue après dispersion des comprimés dispersibles selon l'invention (formulation de l'exemple 1) est plus limpide que la référence (Prozac® comprimé dispersible), comme cela ressort des photos figure 1, qui représentent l'aspect des solutions aqueuses après désagrégation des comprimés dispersibles selon l'invention (les 2 gobelets à gauche sur la photo) et du Prozac^{®} comprimé dispersible (gobelet à droite sur la photo). On constate que les solutions aqueuses obtenues après dispersion du comprimé dispersible selon l'invention sont beaucoup plus limpides que celle obtenue après dispersion du Prozac^{®} comprimé dispersible.

Afin de vérifier et de quantifier cette constatation visuelle, des mesures de turbidité ont été réalisée sur les solutions aqueuses obtenues après désagrégation de la référence (Prozac® comprimé dispersible) ou du comprimé selon l'invention de l'exemple 1.

Les analyses sont respectivement réalisées sur 3 échantillons par lot. Les mesures de turbidité étant enregistrées toutes les 30 secondes pendant 3 minutes.

**Tableau 2 - Mesure des étalons de turbidité**

| **Etalons turbidité** | **Mesure (NTU)** |
|---|---|
| Blanc (sans tube de mesure) | 0,017 |
| Etalon 0NTU | 0,052 |
| Etalon 0-2NTU | 1,53 |
| Etalon 0-20NTU | 18,6 |
| Etalon 0-200NTU | 153 |
| Etalon 200-4000NTU | 1746 |

| | |
|---|---|
| NTU = Nephelometric Turbidity Unit - unité de turbidité néphélométrique | |

**Tableau 3 - Mesure des échantillons**

| **temps (sec)** | **0** | **30** | **60** | **90** | **120** | **150** | **180** |
|---|---|---|---|---|---|---|---|
| **Eau de ville** | 0,316 | 0,260 | 0,254 | 0,209 | 0,204 | 0,201 | 0,194 |
| **référence-1** | 651 | 405 | 269 | 183 | 146 | 114 | 98,3 |
| **référence-2** | 635 | 446 | 275 | 194 | 160 | 133 | 109 |
| **référence-3** | 737 | 456 | 295 | 205 | 187 | 151 | 122 |
| **invention-1** | 103 | 57,1 | 38,7 | 25,0 | 21,5 | 19,5 | 18,0 |
| **invention -2** | 110 | 57,4 | 49,4 | 24,8 | 18,6 | 17,7 | 16,7 |
| **invention -3** | 102 | 72,3 | 37,1 | 25,9 | 20,0 | 17,8 | 15,5 |

Les valeurs mesurées confirment que la solution obtenue après dissolution du comprimé selon l'invention est plus limpide que celle obtenue après dissolution du Prozac^{®} comprimé dispersible.

### Exemple 3 : résultats de bioéquivalence

Les paramètres pharmacocinétiques des comprimés dispersibles selon l'exemple 1, par rapport à la référence (Prozac® comprimé dispersible), pour les deux métabolites fluoxétine et norfluoxetine ont été mesurés.

La bioéquivalence est confirmée. Les valeurs d'AUC (aire sous la courbe) reflètent le taux d'absorption des principes actifs. Les valeurs de Cₘₐₓ (concentration plasmatique maximale) et Tₘₐₓ (temps auquel la Cₘₐₓ est atteinte, pic de concentration) reflètent la vitesse d'absorption des principes actifs. Par rapport à la référence, les comprimés dispersibles selon l'exemple 1 montrent des valeurs de Cₘₐₓ, Tₘₐₓ, et AUC, Kel (constante d'élimination) et T½el équivalentes en fluoxétine et en norfluoxétine.

Les résultats sont résumés dans les tableaux 4 à 7 qui suivent.

**Tableau 4 : Fluoxetine (n=25)**

| Paramètres | Test | | Référence | |
|---|---|---|---|---|
| | Moyenne | C.V. | Moyenne | C.V. |
| Cₘₐₓ (ng/ml) | 26,959 | 20,3 | 25,631 | 18,7 |
| In (Cₘₐₓ) (ng/ml) | 3,2737 | 6,4 | 3,2261 | 6,0 |
| Tₘₐₓ (heures) * | 8,00 | 21,5 | 8,00 | 20,1 |
| AUC_{T} (ng.h/ml) | 1207,541 | 41,8 | 1154,297 | 42,2 |
| In (AUC_{T}) (ng.h/ml) | 7,0254 | 5,4 | 6,9785 | 5,5 |
| AUC_{∞} (ng.h/ml) | 1288,264 | 43,2 | 1226,142 | 43,0 |
| In (AUC_{∞}) (ng.h/ml) | 7,0853 | 5,5 | 7,0351 | 5,6 |
| AUC_{T/∞} (%) | 94,24 | 3,0 | 94,53 | 2,9 |
| Kₑₗ (heures⁻¹) | 0,0209 | 33,6 | 0,0215 | 29,9 |
| T½ₑₗ (heures) | 39,08 | 59,4 | 37,09 | 56,8 |

| | | | | |
|---|---|---|---|---|
| * valeur médiane C.V. = coefficient de variation | | | | |

**Tableau 5 : Fluoxetine**

| Paramètres | Moyenne géométrique des moindres carrés* | | Ratio (%) | Limites de confiance à 90% (%) | |
|---|---|---|---|---|---|
| | Test | référence | | Minimum | Maximum |
| Cₘₐₓ (ng/ml) | 26,422 | 25,178 | 104,94 | 101,76 | 108,22 |
| AUC_{T} (ng.h/ml) | 1125,191 | 1072,760 | 104,89 | 100,81 | 109,13 |
| AUC_{∞} (ng.h/ml) | 1194,712 | 1135,364 | 105,23 | 101,02 | 109,61 |

| | | | | | |
|---|---|---|---|---|---|
| *geometric LS (least square) means | | | | | |

**Tableau 6 : Norfluoxetine (n=25)**

| Paramètres | Test | | Référence | |
|---|---|---|---|---|
| | Moyenne | C.V. | Moyenne | C.V. |
| Cₘₐₓ (ng/ml) | 19,301 | 23,7 | 19,835 | 24,1 |
| ln (Cₘₐₓ) (ng/ml) | 2,9228 | 10,6 | 2,9465 | 11,2 |
| Tₘₐₓ (heures) * | 72,00 | 26,0 | 60,13 | 38,4 |
| AUC_{T} (ng.h/ml) | 5040,085 | 31,0 | 5161,405 | 35,0 |
| ln (AUC_{T}) (ng.h/ml) | 8,4732 | 4,1 | 8,4867 | 4,4 |
| AUC_{∞} (ng.h/ml) | 5372,742 | 35,0 | 5565,446 | 41,1 |
| ln (AUC_{∞}) (ng.h/ml) | 8,5307 | 4,2 | 8,5515 | 4,6 |
| AUC_{T/∞} (%) | 94,51 | 4,4 | 93,85 | 5,1 |
| Kₑ₁ (heures⁻¹) | 0,0060 | 28,3 | 0,0059 | 29,3 |
| T½ₑₗ (heures) | 126,16 | 36,0 | 129,75 | 38,9 |

| | | | | |
|---|---|---|---|---|
| * valeur médiane | | | | |

**Tableau 7 : Norfluoxetine**

| Paramètre | Moyenne géométrique des moindres carrés* | | Ratio (%) | Limites de confiance à 90% (%) | |
|---|---|---|---|---|---|
| | Test | référence | | Minimum | Maximum |
| Cₘₐₓ(ng/ml) | 18,602 | 19,082 | 97,49 | 94,48 | 100,59 |
| AUC_{T} (ng.h/ml) | 4794,299 | 4865,007 | 98,55 | 96,21 | 100,94 |
| AUC_{∞} (ng.h/ml) | 5079,114 | 5193,689 | 97,79 | 95,22 | 100,43 |

| | | | | | |
|---|---|---|---|---|---|
| *geometric LS (least square) means | | | | | |

## Revendications

1. Formulation pharmaceutique appropriée pour la préparation de comprimés dispersibles, par compression directe, constitués de fluoxétine ou d'un sel d'addition acide de celle-ci, à titre de principe actif, en une quantité de 4% à 7,5% en poids par rapport au poids total de la formulation, et d'excipients et coadjuvants appropriés, dont au moins un diluant, un délitant et un lubrifiant, **caractérisée en ce que**
- la fluoxétine est utilisée telle quelle, non revêtue, c'est-à-dire n'ayant pas subi d'étape préalable de revêtement et/ou de mise en forme ou granulation telle que l'obtention de microsphères,
- ledit diluant est un mélange de
- sucres de faible granulométrie, avec une taille moyenne de particules comprise entre 100 µm et 250 µm, et
- sucres de granulométrie moyenne, avec une taille moyenne de particules comprise entre 300 µm et 600 µm,
lesdits sucres étant choisis dans le groupe constitué par le mannitol, le lactose et leurs mélanges.

2. Formulation pharmaceutique selon la revendication 1, **caractérisée en ce que** les particules de sucres de faible granulométrie ont une surface spécifique supérieure à 0,90 m²/g.

3. Formulation pharmaceutique selon la revendication 1 ou 2, **caractérisée en ce que** le diluant est un mélange de
- mannitol avec une taille moyenne de particules comprise entre 150 µm et 220 µm,
- mannitol avec une taille moyenne de particules comprise entre 320 µm et 400 µm.

4. Formulation pharmaceutique selon la revendication 3, **caractérisée en ce que** le diluant est un mélange de
- mannitol avec une taille moyenne de particules comprise entre 150 µm et 220 µm, obtenu par atomisation en lit fluidisé
- mannitol avec une taille moyenne de particules comprise entre 320 µm et 400 µm.

5. Formulation pharmaceutique selon l'une quelconque des revendications précédentes, **caractérisée en ce que** le ratio massique sucres de faible granulométrie : sucres de granulométrie moyenne est de 1.

6. Formulation pharmaceutique selon l'une quelconque des revendications précédentes, **caractérisée en ce que** le délitant est choisi dans le groupe constitué par la carboxyméthylcellulose sodique réticulée, le glycolate d'amidon sodique, et la crospovidone.

7. Formulation pharmaceutique selon l'une quelconque des revendications précédentes, **caractérisée en ce que** le lubrifiant est choisi dans le groupe constitué par le stéarate de magnésium et le talc.

8. Formulation pharmaceutique selon l'une quelconque des revendications précédentes, **caractérisée en ce que** la teneur en diluant est comprise entre 75% et 90% en poids, par rapport au poids total de la formulation.

9. Formulation pharmaceutique selon l'une quelconque des revendications précédentes, **caractérisée en ce que** la teneur en délitant est comprise entre 0,5% et 5% en poids, par rapport au poids total de la formulation.

10. Formulation pharmaceutique selon l'une quelconque des revendications précédentes, **caractérisée en ce que** la teneur en lubrifiant est comprise entre 0,25% et 5% en poids, par rapport au poids total de la formulation.

11. Formulation pharmaceutique selon l'une quelconque des revendications précédentes, **caractérisée en ce que** lesdits excipients et coadjuvants appropriés comprennent en outre les édulcorants et les arômes.

12. Formulation pharmaceutique selon la revendication 11, **caractérisée en ce qu'**elle comprend, à titre d'arôme, des arômes de menthe poivrée et des arômes d'anis.

13. Formulation pharmaceutique selon la revendication 11, **caractérisée en ce qu'**elle comprend, à titre d'édulcorant, la saccharine sodique.

14. Comprimé dispersible obtenu par compression directe de la formulation selon l'une quelconque des revendications précédentes.

## Patentansprüche

1. Pharmazeutische Formulierung, die geeignet ist für die durch direkte Verdichtung erfolgende Herstellung von dispergierbaren Tabletten, welche aus Fluoxetin oder einem Säureadditionssalz von jenem als Wirkstoff in einer Menge von 4 Gew.-% bis 7,5 Gew.-% bezogen auf das Gesamtgewicht der Formulierung und aus geeigneten Arzneimittelträgern und Coadjuvantien, darunter mindestens ein Verdünnungsmittel, ein Spreng- oder Zerfallsmittel und ein Gleitmittel, gebildet werden, **dadurch gekennzeichnet, dass**
- das Fluoxetin als solches, nicht überzogen, eingesetzt wird, d.h. indem es nicht einem vorab erfolgenden Schritt eines Überziehens und/oder einer Formgebung oder Granulation, wie der Gewinnung von Mikrosphären, unterzogen worden ist,
- das Verdünnungsmittel eine Mischung ist von
- Zuckern von geringer Kornklassierung mit einer mittleren Teilchengröße zwischen 100 µm und 250 µm eingeschlossen und
- Zuckern von mittlerer Kornklassierung mit einer mittleren Teilchengröße zwischen 300 µm und 600 µm eingeschlossen,
wobei die Zucker in der aus Mannitol, Lactose und deren Mischungen gebildeten Gruppe ausgewählt sind.

2. Pharmazeutische Formulierung nach Anspruch 1, **dadurch gekennzeichnet, dass** die Zuckerteilchen von geringer Kornklassierung eine spezifische Oberfläche über 0,90 m²/g aufweisen.

3. Pharmazeutische Formulierung nach Anspruch 1 oder 2, **dadurch gekennzeichnet, dass** das Verdünnungsmittel eine Mischung ist von
- Mannitol mit einer mittleren Teilchengröße zwischen 150 µm und 220 µm eingeschlossen und
- Mannitol mit einer mittleren Teilchengröße zwischen 320 µm und 400 µm eingeschlossen.

4. Pharmazeutische Formulierung nach Anspruch 3, **dadurch gekennzeichnet, dass** das Verdünnungsmittel eine Mischung ist von
- Mannitol mit einer mittleren Teilchengröße zwischen 150 µm und 220 µm eingeschlossen, das durch Zerstäubung in einem Wirbelbett erhalten wird,
- Mannitol mit einer mittleren Teilchengröße zwischen 320 µm und 400 µm eingeschlossen.

5. Pharmazeutische Formulierung nach einem der vorangegangenen Ansprüche, **dadurch gekennzeichnet, dass** das Massenverhältnis von Zuckern von geringer Kornklassierung : Zuckern von mittlerer Kornklassierung 1 beträgt.

6. Pharmazeutische Formulierung nach einem der vorangegangenen Ansprüche, **dadurch gekennzeichnet, dass** das Spreng- oder Zerfallsmittel ausgewählt ist in der Gruppe, die aus vernetzter Natriumcarboxymethylcellulose, Natriumstärkeglycolat und Crospovidon gebildet wird.

7. Pharmazeutische Formulierung nach einem der vorangegangenen Ansprüche, **dadurch gekennzeichnet, dass** das Gleitmittel in der aus Magnesiumstearat und Talk gebildeten Gruppe ausgewählt ist.

8. Pharmazeutische Formulierung nach einem der vorangegangenen Ansprüche, **dadurch gekennzeichnet, dass** der Gehalt an Verdünnungsmittel zwischen 75 Gew.-% und 90 Gew.-% eingeschlossen bezogen auf das Gesamtgewicht der Formulierung beträgt.

9. Pharmazeutische Formulierung nach einem der vorangegangenen Ansprüche, **dadurch gekennzeichnet, dass** der Gehalt an Spreng- oder Zerfallsmittel zwischen 0,5 Gew.-% und 5 Gew.-% eingeschlossen bezogen auf das Gesamtgewicht der Formulierung beträgt.

10. Pharmazeutische Formulierung nach einem der vorangegangenen Ansprüche, **dadurch gekennzeichnet, dass** der Gehalt an Gleitmittel zwischen 0,25 Gew.-% und 5 Gew.-% eingeschlossen bezogen auf das Gesamtgewicht der Formulierung beträgt.

11. Pharmazeutische Formulierung nach einem der vorangegangenen Ansprüche, **dadurch gekennzeichnet, dass** die geeigneten Arzneimittelträger und Coadjuvantien außerdem Süßungsmittel und Geschmack- bzw. Aromastoffe umfassen.

12. Pharmazeutische Formulierung nach Anspruch 11, **dadurch gekennzeichnet, dass** sie als Geschmack- bzw. Aromastoff Pfefferminzaromen und Anisaromen umfasst.

13. Pharmazeutische Formulierung nach Anspruch 11, **dadurch gekennzeichnet, dass** sie als Süßungsmittel Natriumsaccharin umfasst.

14. Dispergierbare Tablette, die durch direkte Verdichtung der Formulierung nach einem der vorangegangenen Ansprüche erhalten wird.

## Claims

1. Pharmaceutical formulation suitable for preparing dispersible tablets, by direct compression, consisting of fluoxetine or an acid addition salt thereof, as an active ingredient, in a quantity of 4% to 7.5% by weight in relation to the total weight of the formulation, and suitable excipients and coadjuvants, including at least one diluent, one disintegrant and one lubricant, **characterised in that**
- fluoxetine is used as is, uncoated, i.e. not having undergone a prior coating and/or shaping or granulation step such as obtaining microspheres,
- said diluent is a mixture of
- sugars of small grain size, with a mean particle size between 100 µm and 250 µm
- sugars of medium grain size, with a mean particle size between 100 µm and 600 µm,
said sugars being chosen in the group consisting of mannitol, lactose and mixtures thereof.

2. Pharmaceutical formulation according to claim 1, **characterised in that** the sugar particles of small grain size have a specific surface area greater than 0.90 m²/g.

3. Pharmaceutical formulation according to claim 1 or 2, **characterised in that** the diluent is a mixture of
- mannitol with a mean particle size between 150 µm and 220 µm,
- mannitol with a mean particle size between 320 µm and 400 µm.

4. Pharmaceutical formulation according to claim 3, **characterised in that** the diluent is a mixture of
- mannitol with a mean particle size between 150 µm and 220 µm, obtained by fluidised bed spraying
- mannitol with a mean particle size between 320 µm and 400 µm.

5. Pharmaceutical formulation according to any of the above claims, **characterised in that** the mass ratio of sugars of small grain size: sugars of medium grain size is 1.

6. Pharmaceutical formation according to any of the above claims, **characterised in that** the disintegrant is chosen in the group consisting of cross-linked sodium carboxymethyl cellulose, sodium starch glycolate, and crospovidone.

7. Pharmaceutical formulation according to any of the above claims, **characterised in that** the lubricant is chosen in the group consisting of magnesium stearate and talc.

8. Pharmaceutical formulation according to any of the above claims, **characterised in that** the diluent content is between 75% and 90% by weight, in relation to the total weight of the formulation.

9. Pharmaceutical formulation according to any of the above claims, **characterised in that** the disintegrant content is between 0.5% and 5% by weight, in relation to the total weight of the formulation.

10. Pharmaceutical formulation according to any of the above claims, **characterised in that** the lubricant content is between 0.25% and 5% by weight, in relation to the total weight of the formulation.

11. Pharmaceutical formulation according to any of the above claims, **characterised in that** said suitable excipients and coadjuvants further comprise sweeteners and flavourings.

12. Pharmaceutical formulation according to claim 11, **characterised in that** it comprises, as flavouring, peppermint flavourings and aniseed flavourings.

13. Pharmaceutical formulation according to claim 11, **characterised in that** it comprises, as a sweetener, sodium saccharin.

14. Dispersible tablet obtained by direct compression of the formulation according to any of the above claims.
